Europäisches Patentamt

European Patent Office

Office européen des brevets

(19)

(11) Publication number: **0 293 491**
A1

## (12) EUROPEAN PATENT APPLICATION

published in accordance with Art. 158(3) EPC

(21) Application number: 87907981.2

(22) Date of filing: 03.12.87

Data of the international application taken as a basis:

(86) International application number:
PCT/JP87/00938

(87) International publication number:
WO88/04321 (16.06.88 88/13)

(51) Int. Cl.³: **C 12 N 9/12**
**C 12 N 15/00**

(30) Priority: 03.12.86 JP 288371/86

(43) Date of publication of application:
– 07.12.88 Bulletin 88/49

(84) Designated Contracting States:
BE CH DE FR GB IT LI NL SE

(71) Applicant: MITSUI TOATSU CHEMICALS,
INCORPORATED
2-5 Kasumigaseki 3-chome
Chiyoda-Ku Tokyo 100(JP)

(72) Inventor: MATSUKAGE, Akio
1418, Yamanote 3-chome Meito-ku Nagoya-shi
Aichi 465(JP)

(72) Inventor: YAMAGUCHI, Masamitsu
Kenkousha B-12 1-122, Shimpocho
Chikusa-ku, Nagoya-shi Aichi 464(JP)

(72) Inventor: DATE, Takayasu
19-12, Kikukawa 1-chome Kanazawa-shi
Ishikawa 920(JP)

(74) Representative: Schüler, Horst, Dr.
Kaiserstrasse 69
D-6000 Frankfurt/Main 1(DE)

(54) PROCESS FOR YIELDING MAMMALIAN DNA POLYMERASE -g(b) AND E. COLI STRAIN THUS YIELDED.

(57) A DNA sequence which codes DNA polymerase of animal cell origin having DNA synthetase activity but no nuclease activity is cloned, and the DNA polymerase can be produced in a high yield by incorporating the cloned sequence in a plasmid together with a suitable vector and introducing the recombinant plasmid into host cells such as Escherichia coli to express the code. Since the DNA polymerase can be produced in a high yield, DNA polymerase of animal cell origin can be purified with good efficiency.

EP 0 293 491 A1

0293491

M/3071.7

PCT/JP87/00938

Translation

# DESCRIPTION

Method for Producing Mammalian DNA Polymerase β by
Escherichia Coli, and Escherichia Coli Strain Used therein

## TECHNICAL FIELD

The present invention relates to a DNA polymerase which is very useful not only for fundamental researches in various fields such as biochemistry, molecular biology, genetics and cancer research, but also from the practical and industrial viewpoints in genetic engineering. In particular, it relates to a DNA polymerase having a specific sturcture produced by an animal. Further, the present invention relates to a DNA sequence for coding the DNA polymerase, a recombinant plasmid into which this DNA sequence is integrated, a host cell transformed by using the recombinant plasmid, a method for producing the DNA polymerase by culturing this cell, and a method for separating/purifying the thus produced DNA polymerase.

## BACKGROUND ART

DNA polymerase (deoxynucleoside triphosphorus: DNA deoxynucleotidyltransferase, EC 2.7.7.7) is an enzyme necessary for the synthesis of a DNA as a genetic material in cells and this enzyme is very useful not only for fundamental researches in various fields such as biochemistry, molecular biology, genetics and cancer research, but also from the practical and industrial viewpoints in genetic engineering, and it is definite that the DNA polymerase plays a great role in the development of life scince.

For example, a genetic engineering technique, which has been developed rapidly in recent years, enables cloning an optional gene present in an organism and allowing a suitable host such as Escherichia coli (hereinafter referred to as E. coli) or an animal cell to produce a protein which depends on the gene. Therefore, also in this field, the DNA polymerase is often used as a reagent for the DNA synthesis, and the demand of the DNA polymerase is now being increased.

As the DNA polymerase, there have been heretofore used DNA polymerase I derived from bacteria and a klenow fragment, the so-called klenow fragment enzyme which is a partial decomposition product of the DNA polymerase I, and in particular, the latter klenow enzyme has more often been employed of late.

However, the above-mentioned DNA polymerase I derived from E. coli has DNA lytic enzyme activities, i.e., a 5' → 3' exonuclease activity and a 3' → 5' exonuclease activity in addition to the DNA polymerase activity, and the klenow enzyme also has the 3' → 5' exonuclease activity. In consequence, the DNA synthesis using such enzymes involves the problem that the partial lysis of the synthetic DNA is inevitable.

On the other hand, the DNA polymerases (in eucaryotic cells inclusive of an animal cell, there are plural DNA polymerases which are called DNA poymerase α, β and γ) in cells of animals such as a rat and a human being have no exonuclease activity, as already known, but the amount of these DNA polymerases are, for example, as small as 1/10,000 to 1/100,000 of the total protein. Therefore, a method for extracting/purifying the desired product from the cells can scarcely provide a great deal of the DNA polymerase and is not industrially practical, either.

Among these DNA polymerases derived from animal cells, the DNA polymerase β (hereinafter referred to simply as pol-β) derived from the rat was cloned in the form of cDNA by Zmudzka, B.Z. and Matsukage, who was one of the present inventors, et al (Zmudzka, B.Z. et al, Proc. Natl. Acad. Sci. USA, Vol. 83, p. 5106, July 1986). This cloned rat pol-β gene was composed of 1,197 base pairs, and of these, the code range comprising 954 base pairs decided a protein consisting of 318 amino acid residues.

In various investigations of this cloned rat pol-β gene by the present inventors, its structure have been further researched intensively, and the following fact was found: The amino acid sequence

of pol-β directly separated/purified from a rat and a barnyard fowl was approximately comparable to that of the protein consisting of the 318 amino acid residues coded by this cloned pol-β gene, but when this cloned rat pol-β gene was inserted into a suitable vector to prepare a recombinant plasmid and when the latter was then introduced into E. coli with the intention of trying a genetic expression, it was appreciated that an obtained protein had no rat pol-β activity.

On the other hand, the cloning of the human pol-β is disclosed in SnGupta, D.N. et al, Biochem. Biophys. Res. Commum., 136, p. 341-347, 1986, but according to the similar inspection by the present inventors, it was appreciated that the cDNA obtainted therein was also defective.

DISCLOSURE OF THE INVENTION

The inventors of the present application, taking the above problems into consideration, have conducted a variety of investigations on DNA polymerases derived from animals, and as a result, it has been found that the DNA polymerases are produced in the cells of various animals such as a human being, a mammal, a bird, an amphibian and fish, and it has been concluded that the DNA polymerase derived from the animal cell can be produced in large quantities by first cloning the gene for coding the DNA polymerase of the animal cell, forming a recombinant plasmid, introducing this plasmid into a suitable host cell, and then expressing the gene. The present invention has been completed by further intensive researches on the basis of the above-mentioned knowledge.

That is, an object of the present invention is to provide a DNA polymerase derived from an animal cell and having no nuclease activity which is very useful not only for fundamental researches in various fields such as biochemistry, molecular biology, genetics and cancer research, but also from the practical and industrial viewpoints in genetic engineering.

Another object of the present invention is to provide a method for producing the DNA polymerase derived from an animal cell in a high yield, a DNA polymerase producing strain which is useful in this method, and a method for separating/purifying the DNA polymerase in a high yield from bacteria cells prepared by culturing the strain.

In order to accomplish the above-mentioned objects, the present inventors have paid much attention to the DNA polymerase β among the above-mentioned three kinds of DNA polymerases, because the DNA polymerase β comprises a single chain of 40 kDa (kilodalton) and has the simplest structure. In addition, they have paid more attention to the fact that

the DNA polymerases β derived from a rat and a human being have no nuclease activity, and they have further investigated structures and functions of these DNA polymerases β.

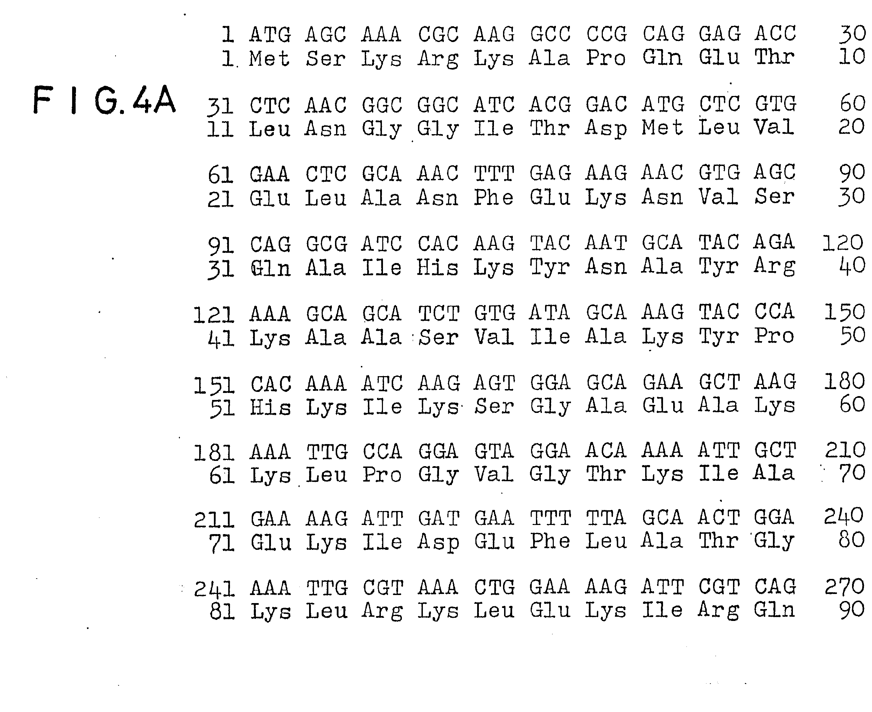

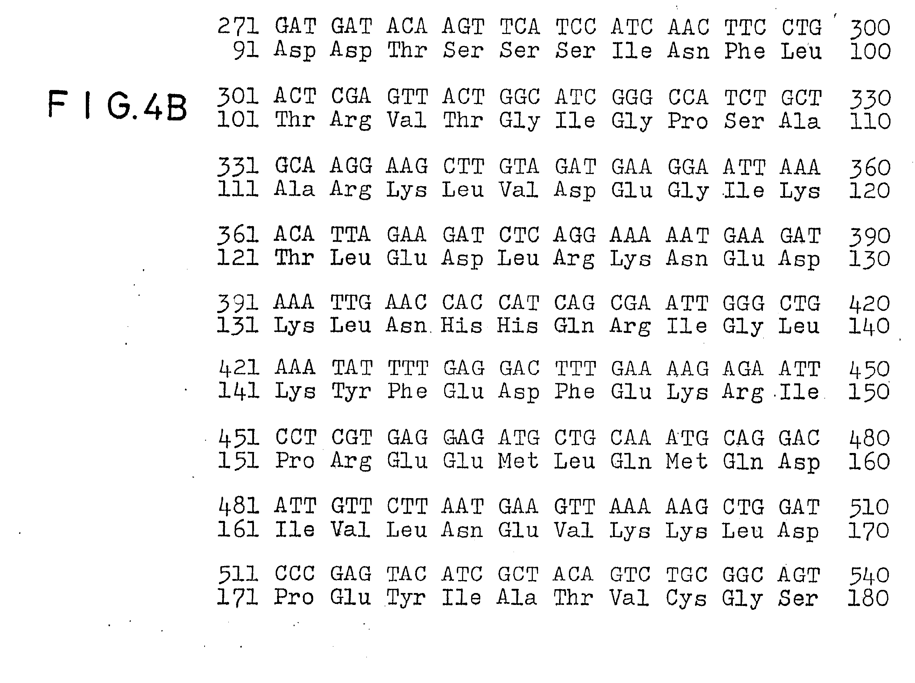

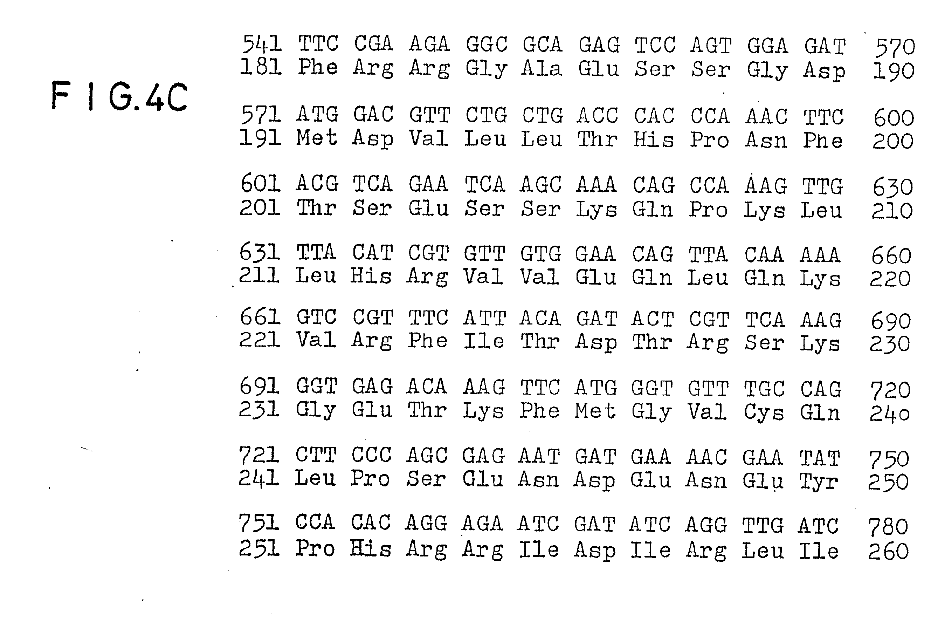

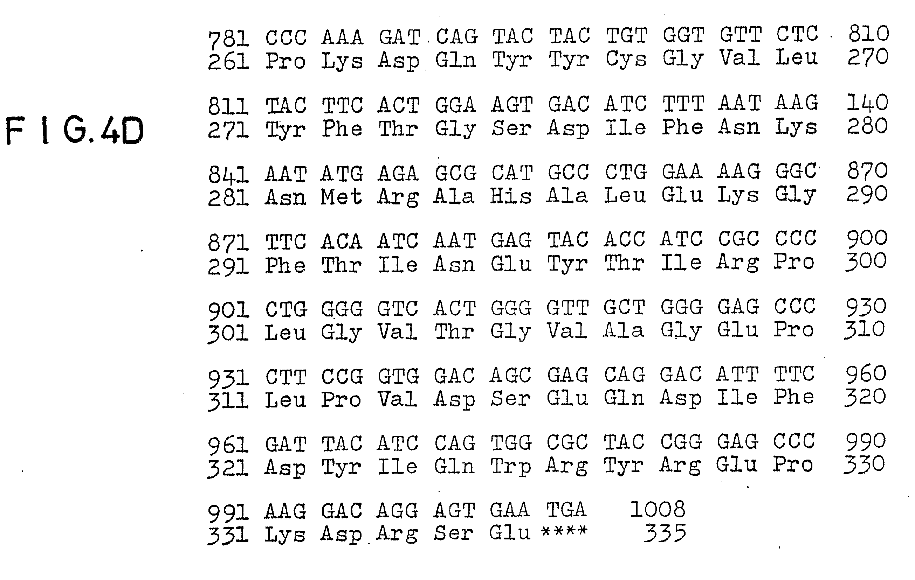

The inventors more deeply studied the problem that the protein produced by the expression of the above cloned rat pol-β gene in E. coli formed by Matsukage et al had no pol-β activity. That is, the rat pol-β gene was separated from the chromosome of a rat in a conventional manner, and its DNA sequence was inspected. As a result, it was found that the amino acid sequence which was dependent on the cloned rat pol-β gene was defective to constitute the polypeptide structure of the DNA polymerase, i.e., it was lacking in 17 amino acid residuals on the side of the N-terminal side, and that its cause was that a defective portion of 9 bases (the beginning 9 bases of the base sequence in Fig. 4) occurred in the cloned DNA sequence.

The present inventors established a method for compensating the defective or incomplete portion, and they succeeded in the synthesis of a DNA sequence for coding a polypeptide having the pol-β activity. In addition, they established another method for preparing the rat and human DNA polymerases consisting of 335 amino acid residuals in a high yield by means of a genetic engineering technique, and finally the present invention has been completed.

The present invention to accomplish the above objects contains the pol-β derived from an animal cell and comprising the amino acid sequence described in claim 1, a DNA sequence coding for the pol-β, a recombinant plasmid formed by using the DNA sequence, transformed cells carrying the recombinant plasmid, a method for producing the pol-β by using the transformants and a method for purifying the pol-β thus produced.

According to the present invention, the DNA sequence for coding the DNA polymerase derived from an animal cell which has a high DNA synthesis enzyme activity and a less nuclease activity can be cloned, and it can be integrated into a plasmid together with a suitable vector in order to form a recombinant DNA, which is then introduced into a host cell such as E. coli or the like, with the result that the DNA polymerase is expressed. In such a way, the DNA polymerase can be produced in a high yield of, for example, 20%.

Further, according to the present invention, the DNA polymerase produced in the host cells can be separated/-purified in a pure form in a high yield from the cultivation system of the host cells, which enables the mass production of the pure DNA polymerase.

Above all, the present inventors have investigated the DNA polymerases derived from the cells of animals such as a fowl, a rat and a human being, and they have found that the amino acid sequence of the rat is homologous to that of the human being as much as 95%. In consequence, they have first intended the cloning and the expression of the rat DNA polymerase β gene by the utilization of a genetic engineering technique, and they have succeeded in it. In addition, the rat DNA polymerase β obtained in this manner is uniform and has a sufficiently high enzyme activity. This successful result supports that the present invention enables the expression of the DNA polymerase in the cells of hosts such as a fowl and a human being other than the rat by the genetic engineering technique and permits the mass production of the DNA polymerase in the pure form.

In addition, although the DNA polymerase I derived from E. coli and the klenow enzyme which have often heretofore been used possess the nuclease activity, the DNA polymerase β obtained by the present invention has no nuclease activity. Therefore, in the DNA synthesis by the use of the DNA polymerase which the present invention provides, the 3' terminal thereof can be completed. In consequence, the DNA polymerase of the present invention can be directly applied to the DNA synthesis process in which any nuclease activity is not required or is not desired, and it can be mass-produced in the pure form. It is fair to say that the present invention can sufficiently meet the increasing demand for the DNA polymerase.

## BRIEF DESCRIPTION OF THE DRAWINGS

Fig. 1 shows a construction process of the plasmid for expressing the DNA polymerase of the present invention;

Fig. 2 is a schematic view showing an insertion direction of the DNA fragment comprising a part of the rat DNA polymerase β gene in the plasmid obtained in paragraph (1) of Example 1;

Fig. 3 illustrates a site specific mutation induction process in the present invention; and

Fig. 4 shows a DNA sequence for coding the rat DNA polymerase β obtained in Example 1 of the present invention, and an amino acid sequence coded thereby. It should be noted that these sequences are continuous over Figs. 4A to 4D and each of them constitutes one sequence.

In the drawings, P., O., SD and MCS represent promoter, operator, Shine Dalgarno sequence and multicloning site (polylinker site), respectively. F is the part moving at a high rate and S is the part moving at a low rate, in an electrophoresis.

## BEST MODES FOR CARRYING OUT THE INVENTION

The DNA sequence for coding the DNA polymerase derived from an animal cell of the present invention can be prepared by cloning the DNA sequence for coding the DNA polymerase contained in the DNA polymerase gene of various animal cells which produce the DNA polymerase.

- 10 -

0293491

The examples of these animals used herein include a salmon, a trout, a frog, a fowl, a mice, a rat, a woodchack, a pig, a sheep, an ox, a monkey and a human being.

Of these animals, cells of animals such as a rat and a human being are preferred, as described above, for. the purpose of obtaining the DNA polymerase having no nuclease activity.

A useful method for cloning the DNA for coding the desired DNA polymerase is such a method as described in Example 1 which has been established by the present inventors and which will be discussed hereinafter.

That is, the DNA (doulbe chain) which has been produced by cloning the DNA polymerase gene in the conventional manner contains the defective or incomplete section, as described above. The repair of the defective portion may be carried out as follows: In the first place, a single chain DNA fragment is synthesized. This DNA fragment has a base sequence which is complementary to the portion to be supplemented to the double helix DNA, and it is further provided, on the upstrem and downstream sides of the above base sequence thereof, with base sequences which are complementary to the upstream and downstream sides of the defective portion on the double helix DNA. Next, the double helix DNA is split into single chains, and the one single chain thereof having 'the defective portion is then reacted with the previously prepared DNA fragment including the base sequence complementary to the supplementary part.

Since both the upstream and downstream sides of the DNA fragment are complementary to those of the single chain having the defective portion, the DNA fragment is combined with the single chain having the defective portion, in positions on both the sides thereof, so that the combined portions takes a double helix structure.

Afterward, a treament is applied so that a site specific mutation may occur in the joined section between the DNA fragment and the single chain having the defective portion. As a result, a nucleotide sequence containing a complementary base sequence is synthesized in the defective portion between the joined portions of the single chain, thereby obtaining the double helix DNA having the desired complementary base sequence thus supplemented in the section in which the defective section has been present before.

Such a manner for repairing the defective portion on the single chain DNA by the occurrence of the site specific mutation thereon may be suitably applied not only to the DNA sequence having the above-mentioned defective or imperfect portion but also to the DNA having a section which is homologous to a predetermined DNA sequence, and in the latter case, the desired DNA may be obtained by a suitable supplement to the insufficient base sequence section thereon. For example, even if a desired DNA sequence cannot be cloned in a perfect form, the above-mentioned manner can repair a defective portion on the DNA. This convenient manner is called the mismatch primer process.

Although Example 1 given hereinafter describes the cloning of a DNA sequence for coding a rat pol-β derived from a rat, the mismatch primer process can also be applied to the cloning of the DNA sequences derived from the above-mentioned various animals which can produce DNA polymerases.

The DNA sequence for coding the DNA polymerase which has been cloned in such a manner is that which is newly constructed by the present inventors, and in consequence, the formation of the recombinant DNA for producing the DNA polymerase has become possible and the mass production of the DNA polymerase has also become possible.

Next, reference will be made to a production process of the DNA polymerase by the use of the DNA sequence for coding the DNA polymerase of the present invention.

The examples of host cells which can be used in the production process of this DNA polymerase include cells of bacteria such as E. coli, Actinomycetes and Bacillus subtilis, and micoorganisms such as yeast, a salmon, a trout, a fowl, a mice, a rat, a woodchuck, a sheep, an ox, a monkey and a human being.

As the vector into which the DNA sequence for coding the DNA polymerase of the present invention is introduced, any type thereof can be used in so far as its replication is possible in the host cell and a DNA polymerase expression is possible in the host cell for the DNA polymerase. Examples of such vectors include a vector having a lac operon promoter, pKK 322 vector having tac promoter, and a vector having a trp operon promoter.

Further, if necessary, the vector may include a marker for indicating the resistance to ampicillin, tetracycline, kanamycin and the like, in addition to such a promoter as mentioned above.

- 12a -

In the case that there is used, as the vector, a plasmid having the characteristics that the DNA of the plasmid is split into two single chains by adding a phage such as pUC118 (made by Takara Shuzo Co., Ltd.), the vector can be immediately combined with a synthesized oligonucleotide primer containing a site specific mutation region, and therefore, the desired double helix DNA can be obtained in which substitution, insertion, deletion or the like is given by the mismatch primer process. The thus obtained DNA is introduced into a suitable host, whereby a protein having a new physiological activity can be expressed. In addition, other vectors such as pTZ18R and pTZ19R (made by Pharmacia Co., Ltd.) can also be used for these purposes, these vectors also having the characteristics that its DNA is split into single chains.

The DNA sequence for coding the DNA polymerase of the present invention can be integrated into such a type of vector in accordance with an ordinary procedure by the utilization of a suitable restriction enzyme cleavage sites and, if necessary, by the use of a linker, so that a recombinant DNA for producing the DNA polymerase can be prepared.

The recombinant DNA prepared in Example 1 which will be described hereinafter is particularly suitable for the production of the DNA polymerase, and it is that which the

- 13 -

0293491.

present inventors have constructed newly.

In order to carry out the transfection of the recombinant DNA, which has the DNA sequence for coding the DNA polymerase of the present invention, into a host cell, there can be employed a calcium chloride method, a calcium phosphate method, a protoplast fusion method or the like.

The host cell into which the recombinant DNA is brought in this way is then transformed and cultured in order to produce the desired DNA polymerase.

For example, as described in Exmaple 2 given hereinafter, the DNA polymerase was produced in a yield of 19.3% based on the total protein in the bacteria cells in accordance with the method of the present invention.

Moreover, the DNA polymerase produced in the host cells in accordance with the above-mentioned method can be purified by the procedure which will be described in detail.

The present inventors have researched on the structure and function of known various DNA polymerases, but the DNA polymerase is present in a trace amount in the animal cell and its separation and purification are very difficult. Neverthless, the present inventors succeeded in the acquisition of the single DNA polymerase by combining about 10 steps.

Concretely speaking, in view of the fact that the DNA polymerase is a basic protein and removes nucleic acids such

- 13a -

as contaminated DNAs, the present inventors have concluded that it is necessary to treat a mixture such as a bacteria cell extract solution containing the DNA polymerase with an anion exchange carrier such as DEAE cellulose.

Next, the fraction of the mixture which was not adsorbed by the anion exchange carrier was adsorbed by an acidic cellulose phosphate carrier, and from the adsorbed fraction, the DNA polymerase was separated/purified by a difference between salt concentrations of eluates.

In the last step, the DNA polymerase was further purified by an affinity chromatograph having a DNA column.

In this separation/purification process, a variety of operations inclusive of a gel filtration were carried out in addition to the above main steps, thereby separating/purifying the extreme trace amount of the DNA polymerase derived from the animal cell.

In contrast, in the present invention, since the animal DNA polymerase is produced in large quantities in bacteria or the like, a crude product containing the DNA polymerase can be obtained abundantly. The present inventors have omitted several steps other than the above-mentioned main three steps, i.e., have combined the especially effective steps alone in order to establish a novel purification process in which the operation steps are simplified remarkably.

The examples of the anion exchange carriers used in the first main step include DEAE cellulose, TEAE cellulose, DEAE Sephadex, QAE Sephadex, Sephacel and Dowex-1.

. The examples of the acidic carriers (cation exchangers) used in the second main step include cellulose phosphate, CM cellulose, SE cellulose, CM Sephadex, hydroxy apatite and Dowex-50.

As the materials for the affinity chromatograph used in the third main step, DNA cellulose, a blue agarose or the like may be used.

In the separation/purification process of the present invention, an ammonium sulfate fraction method and an organic solvent method may be utilized. Further, subsequent to the third main step, there may be employed a gel filtration method using, for example, Sephadex G150 and a purification step by means of the affinity chromatography using the DNA cellulose.

Next, the typical example of the separation/purification process in the present invention will be described.

In the first place, the host cells in which the DNA polymerase has been produced are washed with TME [50 mM of tris-HCl (pH 7.6), 10 mM of $MgCl_2$ and 1 mM of EDTA], and the cells are stored at -80°C, if the amount thereof is large.

The cells are then suspended in an extracting solution [50 mM of tris-HCl (pH 7.6), 0.1 mM of EDTA, 1 mM of dithiothreitol (DTT), 10% glycerin, 0.5 M of KCl and 1 mM of phenylmethylsulfonyl fluoride (PMSF)] in an amount 5 times as much as that of the cells, and an ultrasonic wave is applied to the solution for 1 minute in order to destroy the cells. The solution is further treated by a centrifuge (12,000 xg, for 20 minutes) to remove a precipitate therefrom, thereby obtaining a crude extract of the cells.

For the preparation of this crude extract, a method of using lysozyme and/or a neutral surface active agent may be suitably used, and other various methods also are usable.

Afterward, a PC solution [50 mM of tris-HCl (pH 7.6), 0.1 mM of EDTA, 1 mM of DTT and 10% of glycerin] is added to the crude solution so as to dilute the latter about 1.5-fold. This dilution is such that the solution is treated smoothly by a DEAE cellulose column.

A KCl concentration in the diluted crude extract is adjusted to 0.4 M and then is caused to pass through the column filled with DEAE cellulose (made by Pharmacia Co., Ltd.), so that nucleic acid components contained in the diluted crude extract are adsorbed by the DEAE cellulose, so that these components are removed from the crude extract.

The PC solution having the above-mentioned composition is added to the fraction (containing the DNA polymerase) which has not been adsorbed by the column, so as to dilute the fraction 2 to 3-fold, and a concentration of KCl is then

adjusted to 0.2 M.

A column filled with cellulose phosphate (made by Sigma Co., Ltd.) is then treated with a PC solution containing 0.2 M of KCl to equilibrate the cellulose phosphate. Afterward, the above prepared solution is caused to pass through the column, and the latter is further washed sufficiently with the PC solution containing 0.2 M of KCl.

This operation permits adsorbing, on the column, most of the DNA polymerase derived from the DNA sequence for coding the DNA polymerase of the present invention. In this step, the DNA polymerase derived from the host cell is not adsorbed on the column but is eluted out, with the result that the latter DNA polymerase is separated from the desired DNA polymerase.

The protein adsorbed on the column can be eluted by the use of a straight concentration gradient of 0.2 to 0.7 M KCl, and a single peak of the DNA polymerase activity is obtained in the vicinity of about 0.4 M of KCl. The fractions having the high DNA polymerase activity are collected and then diluted with the PC solution having the above-mentioned composition. Afterward, KCl and glycerin concentrations in the solution are adjusted to 0.15 M and 20%, respectively.

Next, in accordance with Litman's method (Litman, R.N., J. Biol. Chem., 243, 6222 to 6233, 1969), the thus prepared solution is caused to pass through a DNA cellulose column made from a modified calf thymus DNA (made by Washington Inc.). After the column has been washed sufficiently with the above-mentioned PC solution, the adsorbed DNA polymerase is eluted from the column by the use of 0.6 to 0.7 M of a KCl-PC solution (containing 25 vol% of glycerin). At this time, a coincident single peak of both the DNA polymerase activity and a protein production can be procured, and the fraction at this single peak is recoved, thereby obtaining the purified DNA polymerase product. For the purpose of preserving this purified product stably, it is preferred that glycerin is added to the product so that the concentration of the glycerin may be at a level of 50% and its storage temperature is kept up at -20°C.

The purified poduct, if necessary, may be additionally subjected to the combination step of a gel filtration and an affinity chromatography which will be described below.

A colunm is filled with, for example, Cephadex G150 and is then equilibrated with a 0.15 M KCl-PC solution, and the purified product obtained through the above-mentioned third main step is injected into the column. Eluation is then carried out with 0.15 M KCl-PC solution to dispense an eluate, and for each fraction, a DNA polymerase activity is measured. Next, the fractions having a high DNA polymerase activity are collected, and the resulting solution is then injected into the DNA cellulose column. Further, sufficient washing is performed with a 0.15 M KCl-PC solution, and then the components adsorbed on the column are eluted with 15 ml of a 0.15 to 0.7 M KCl by the utilization of its straight concentration gradient (in a PC solution). Afterward, the fractions having the high DNA polymerase activity are collected, thereby obtaining a purified product. In this case, the DNA polymerase can be eluted with KCl at a concentration of about 0.4 M.

The thus obtained pure product contains a uniform DNA polymerase and has the same enzyme activity as in a natural DNA polymerase, and the specific activity of the product is also as high as in the natural one.

### EXAMPLE

Now, the present invention will be described in detail in accordance with examples, but it should not be limited to these examples and can take various modifications.

Example 1

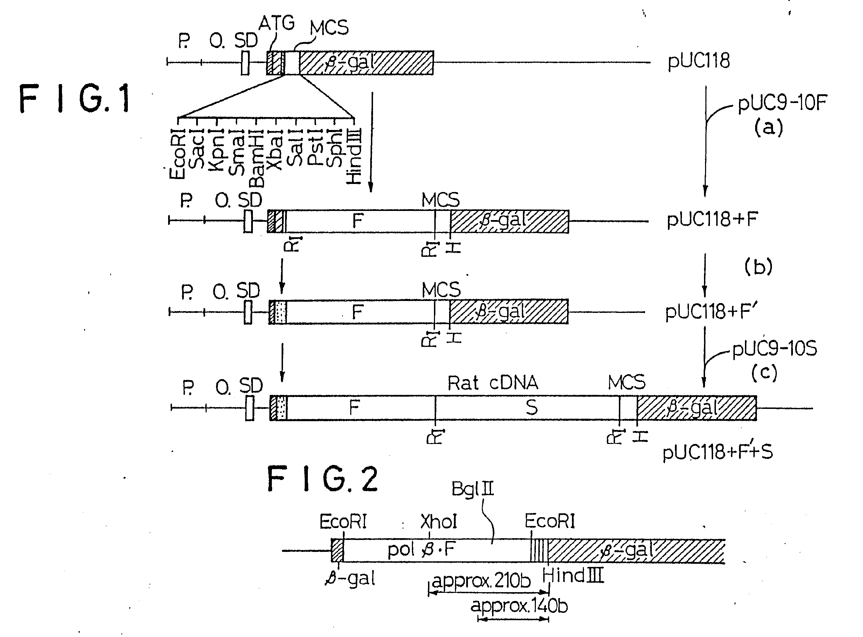

(Production of plasmid for rat pol-β expression in accordance with process shown in Fig. 1)

(1) Preparation of first half of rat pol-β structural gene

[Process shown Fig. 1 (a)]

In the first place, 10 μg of a plasmid pUC9-10F having cDNA were cut with a restriction enzyme EcoRI. The cDNA used herein included a DNA sequence for coding half a section (lacking the first 9 bp including the initiation codon for 3 amino acid residual groups) on the N-terminal side of a rat pol-β which was obtained by A. Matsukage et als' method (Proc. Natl. Acad. Sci. USA, Vol. 83, p. 5106, July 1986).

The restriction enzymes used in this example were T4 ligase and T4 Kinase which were both made by Nippon Jean Co., Ltd. The reactions regarding this restriction enzymes were carried out in ordinary manners, unless otherwise specified.

The defective portion in the DNA sequence for coding half the section on the N-terminal side of the rat pol-β in the plasmid was determined in accordance with the aforesaid process by the present inventors.

After the reactions had been over, the reaction solution was subjected to the electrophoresis using 0.6% of a low-gelling temperature agarose gel (made by BRL Inc., LMP Argarose) in order to separate a fragment of 440 bp from the mixture of DNA fragments. Afterward, the gel portion containing this 440 bp fragment was cut out and transferred into a plastic tube. Heating was then performed up to 65°C so as to melt the gel, and a TE buffer solution [10 mM tris-HCl (pH 8.0) and 1 mM EDTA] saturated phenol was added into the tube until about 1/3 of the tube volume had been reached, followed by shaking it well. In addition, the solution was treated by a centrifuge at 12,000 rpm for 5 minutes to obtain an aqueous phase.

From the latter, a remaining phenol was removed by repeating an ether treatment thrice, followed by an ethanol precipitation treatment to recover the DNA.

On the other hand, 3 µg of pUC118 vector (made by Takara Shuzo Co., Ltd.) was cut perfectly with the restriction enzyme EcoRI and was then treated with an alkali phosphatase (made by Boehringer Mannheim A.G.).

Next, 1 µg of the linear pUC118 which had been cut by the EcoRI was reacted with the previously recovered 440 bp DNA fragment at room temperature for 2 hours in the presence of $T_4$ ligase (500 units).

After the completion of the reaction, a part of the reaction product was introduced into E. coli MV 1304 strain (made by Takara Shuzo Co., Ltd.) in accordance with the calcium process, and the E. coli was cultured in the L-broth having the following composition. Afterward, the transformed strain was chosen by the use of an ampicillin (50 µg/ml) plate [which had been prepared by adding 1.5% of agar (Bacto Agar, made by Difco Co., Ltd.) to the L-broth and further adding thereto ampicillin (made by Meiji Seika Kaisha, Ltd.)].

L-Broth Composition:

|  |  |
|---|---|
| Bactotryptone (Difco Co., Ltd.) | 10 g |
| Yeast Extract (Difco Co., Ltd.) | 5 g |
| NaCl | 5 g |

Glucose                                    1 g

Water                                      1 ℓ

From the colonies showing ampicillin resistance, 12 colonies were chosen and shake-cultured separately in 1.5 ml of the L-broth having the above-mentioned composition, and minilysates were prepared from the cultured cells. Then, the minilysates were cut by the combination of the restriction enzymes Hind III and Xho I, and by the other combination of Hind III and Bgl II to prepare two DNA fragments. They were separately subjected to a 1% agarose electrophoresis with the intention of inspecting the constitution of the plasmid contained in the transformed strain.

On the basis of this result, there were chosen three colonies (#7,#8,#9) containing the plasmid in which the DNA frangment (440 bp) containing a part of the rat pol-β gene derived from the plasmid pUC9-10F was inserted in the same direction as in a β-galactosidase (β-gal) gene, as shown in Fig.2.

(2) Preparation of single chain DNA

The three colonies #7, #8 and #9 which had been chosen in the above paragraph (1) were separately shake-cultured in a culture medium in which 50 μg/ml of ampicillin was added to the 2XYT culture medium having the following composition. When a bacteria cell concentration in each culture medium had reached a level of $OD_{600}$ = about 0.1, $1 \times 10^9$ p.f.u. of helper phage M13K07 (Takara Shuzo Co., Ltd.) was added to

- 22 -                                    0293491

each culture medium, and the shake culture was further
continued overnight.

Composition of 2XYT Culture Meidum:

|  |  |
|---|---|
| Bactotryptone (Difco Co., Ltd.) | 32 g |
| Yeast Extract (Difco Co., Ltd.) | 20 g |
| NaCl | 10 g |
| Water | 1 ℓ |

After the completion of the culture, the bacteria cells
were removed from each culture medium by means of a
centrifuge (12,000 rpm, for 10 minutes), and 0.3 g of NaCl
and 0.2 g of polyethylene glycol 6000 (trade name, made by
Wako Pure Chemicals Co., Ltd.) were added to the resulting
supernatant liquid. The solution was then allowed to stand
for 2 hours at 4°C in order to condense the phage particles.

Next, the condensed phage particles in the supernatant
liquid were collected by the centrifuge (12,000 rpm, for 10
minutes), and each sample was suspended in 300 µl of a TE
buffer solution. Afterward, 200 µl of a mixed solution of
phenol and chloroform (1:1) was added to each suspension,
and the suspension was then shaken well to remove proteins
therefrom. An ether treatment was then carried out thrice
to remove the remaining phenol from the suspension, and in
the final step, an ethanol precipitation treatment was
perfomred to obtain a purified product. The yield of the
purified product in each case was 4.5 µg in terms of

the suspension.

When this purified product was subjected to an agarose gel electrophoresis, it was confirmed that the product was the single chain DNA comprising one chain of the plasmid in which the DNA frangment (440 bp) containing a part of the rat pol-β gene derived from the plasmid pUC9-10F obtained in the preceding paragraph (1) was inserted in the same direction as a β-galactosidase (β-gal) gene.

(3)    Induction of mutation by oligodeoxynucleotide [Process shown in Fig. 1 (b)]

The single chain DNA obtained in the preceding paragraph (2) intactly had a defective portion of 9 bases, i.e., 3 amino acid residues from the initiation codon of the DNA sequence for coding half the section on the N-terminal side of the rat pol-β of the 440 pb DNA fragment obtained in the preceding paragraph (1).

This defective portion was repaired as follows:

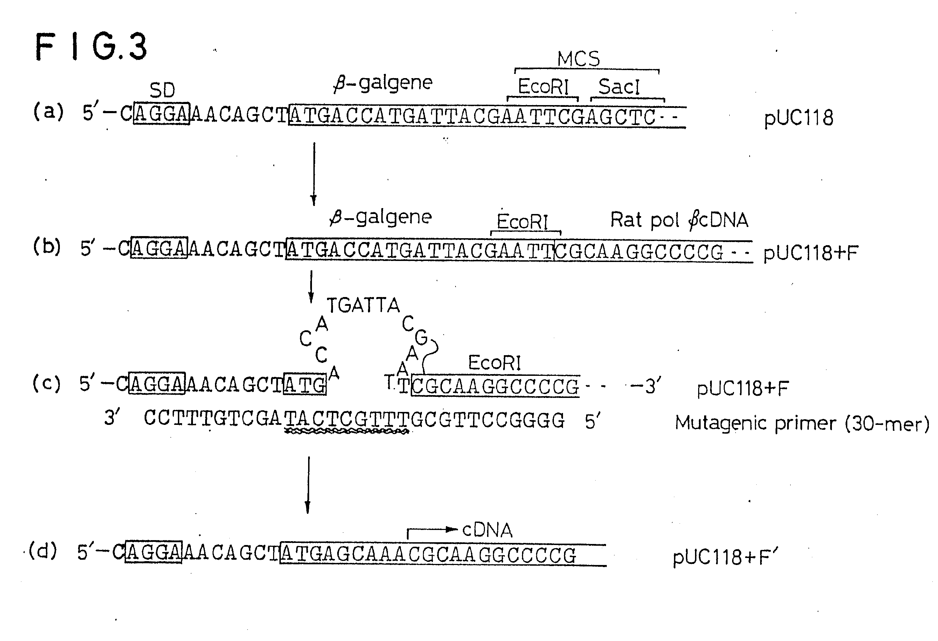

In the first place, an oligodeoxynucleotide (30 bases) was synthesized by the use of Genet which was available from Nippon Zeon Co., Ltd. This oligodeoxynucleotide had a DNA sequence corresponding to the above defective portion shown by the double wavy lines in Fig. 3(c); a DNA sequence, which was located on the 3' side of the DNA sequence corresponding to the defective portion and which was complementary to the base sequence on the upstream side of the initiation codon of the β-gal gene; and another DNA sequence, which was located on the 5' side of the DNA sequence corresponding to the defective portion and which was complementary to the first 11 bases located on the downstream side of the defective portion (which was composed of 9 bases including the initiation codon).

Next, this oligodeoxynucleotide was phosphorylated at the 5' position thereof. This phosphorylation was carried out by reacting (37°C, for 30 minutes) 35 ng of oligodexoynucleotide with 16 units of $T_4$ Kinase (Boehringer Mannheim A.G.) in an aqueous solution containing 50 mM of tris-HCl (pH 7.9), 6 mM of $MgCl_2$, 10 mM of β-mercaptoethanol and 0.5 mM of ATP, and then performing a heat treatment at 100°C for 30 seconds to deactivate $T_4$ Kinase and to thereby terminate the reaction.

After the completion of the reaction, 2 μl of the reaction solution (containing 7 ng of phophorylated oligodeoxynucleotide) was mixed with 2 μl of the TE buffer solution having the above-mentioned composition and containing 0.9 μg of the single chain DNA derived from the colony #8 obtained in the preceding paragraph (2). Another buffer solution for annealing was further added thereto so as to bring the total volume to 10 μl, and the solution was then gotten into a capillary and sealing was made. In this case, the composition (at the final concentration) of the buffer solution for annealing was composed of 30 mM of tris-HCl (pH 7.9), 6 mM of $MgCl_2$, 120 mM of NaCl and 10 mM of β-mercaptoethanol.

Afterward, the reaction solution in the capillary was first subjected to a heat treatment at 100°C for 1 minute, and its temperature was caused to fall to 60°C and further to 28°C gradually over 3 hours.

The capillary was then opened to take out the reaction solution, and thereto were added 0.8 μl of an aqueous solution (made by Yoshitomi Pharmaceutical Industries, Ltd.) containing 10 mM of dATP, dGTP, dCTP and TTP, 0.8 μl of an aqueous solution (made by Sigma Co., Ltd.) containing 10 mM of ATP, 4 units of klenow enzyme (made by Boehringer Mannheim A.G.) and 5000 unites of $T_4$ ligase and reaction was then performed at 28°C for 2 hours.

The reaction was terminated by adding a mixed slution of phenol and chloroform (1:1) to the reaction solution.

After the completion of the reaction, an ether treatment was carried out for the reaction solution thrice, and a part of the solution was injected into E. coli HB101 (which was available from Dr. Dan S. Ray, UCLA Molecular Bilogoy Laboratory) in accordance with the calcium chloride method.

In this case, the single chain DNA derived from the colony #8 obtained in the preceding paragraph (2) was replaced with the single chain DNA derived from the colony #9 and a similar procedure was taken, and the finally obtained reaction solution was introduced into the E. coli HB101 by the calcium chloride method.

(4)  Detection of recombinant

The two kinds of <u>E. coli</u> HB101 into which the reaction solution had been introduced in the preceding paragraph (3) were cultured separately in the above-mentioned L-broth culture medium, and the colonies showing ampicillin resistance were chosen by the ampicillin plate used in the previous paragraph.

Next, the colonies having the desired plasmid containing a DNA sequence for coding half the section on N-terminal side of the rat pol-β were chosen from the above screened colonies by a colony hybridization method which would be described hereinafter.

That is, a filter (made by MiliporeCo., Ltd., HA type, 0.45 μm, diameter 82 mm) was put on the ampicillin plate, and bacteria cells were transferred from the colonies on the plate to the filter.  The surface of the filter on which the bacteria cells were adsorbed was turned up and put on each filter paper through which the undermentioned solution had been allowed to penetrate for a predetermined period of time.

(1)  0.5 N NaOH for 7 minutes,

(2)  0.5 M tris-HCl (pH 7.4) for 2 minutes

(3)  0.5 M tris-HCl (pH 7.4) for further 2 minutes

(4)  0.5 M tris-HCl (pH 7.4) containing 1.5 M NaCl for 5 minutes.

Next, 50 ml of the solution (4) mentioned above was caused to pass through the filer and 50 ml of chloroform was further caused to pass through the filer, and the filter was then dried. In addition, baking was carried out at 80°C for 1.5 hours in vacuo, thereby fixing the DNA thereon.

The filter on which the DNA was fixed was dipped into the mixed solution of x10 Denhardt solution (x1 Denhardt solution: 0.02% of polyvinylpyrrolidone, 0.02% of Ficoll 400 and 0.02% of calf serum albumin), 100 µl/ml of modified salmon spermatozoon DNA, x4 SSC (x1 SSC: 0.15 M of sodium chloride and 0.015 M of trisodium citrate; adjusted to pH 7.4 with hydrochloric acid) and 0.5% of Triton X-100 in order to carry out hybridization at 65°C for 2 hours. Afterward, the solution was drawn off, and the filter was further dipped into another solution (total volume 2 ml) of x4 SSC, x10 Denhardt solution and 0.5% Triton X-100 in order to carry out hybridization at 58°C for 10 hours, the aforesaid solution containing 30 ng of synthesized oligodeoxynucleotide, marked with $(^{32}P-\gamma)$ATP 10 µCi, as a probe having the same base sequence as the oligodeoxynucleotide used in the preceding paragraph (3).

After the completion of the reaction, the filter was washed thrice with 60 ml of x1 SCC at 50°C for 5 minutes. Then, the filter was dried and brought into contact with an X-ray film (Fuji Dental Panorama NIF type).

As a result, it was found that about 15% of the colonies into which the reaction solution derived from #8 was introduced and which exhibited the ampilillin resistance had the DNA combining strongly with the probe, and that about 30% of the colonies into which the reaction solution derived from #9 was introduced and which exhibited the ampilillin resistance had the DNA combining with the probe.

In the colony which is the origin of the DNA having the positive reactivity, there may still remain the variant (in which the above-mentioned defective portion has been repaired) and the nonvariant (in which the defective portion has not been repaired), and therefore it is necessary that the existence of the variant in the colony which is the origin of the DNA having the positive reactivity is confirmed, that the plasmid is then separated from the colony and is introduced into E. coli again, and that the strain having the variant alone is chosen.

Hereupon, 9 colonies were selected from the colonies into which the reaction solution derived from #8 was introduced and which showed the ampicillin resistance and the positive reactivity, and 4 colonies were selected from the colonies into which the reaction solution derived from #9 was introduced and which showed the ampicillin resistance and the positive reactivity. Each colony was shake-cultured in 1.5 ml of the above-mentioned L-broth for 14 hours, and a minilysate was prepared form each sample in an ordinary manner, was then cut by EcoRI, and was subjected to a 1% agarose electrophoresis.

In this case, the EcoRI cut positions (RI) are two, as shown in Fig. 1 (b) where constitutions of the plasmid before and after the process are illustrated, but in the variant, the cut position decreased to one, and hence it can be detected with ease.

The colonies (#8-2 and #8-7) derived from #8 and the colonies (#9-12) derived from #9 which contained a relatively great deal of the variant were chosen, and the respective minilysates were prepared in an ordinary manner and were then introduced separately into E.coli JM 109 (Takara Shuzo Co., Ltd.). They were then cultured, and transformed strains were selected therefrom on an ampicillin plate.

The transformed strains #8-7-7 and #9-12-16 thus obtained were cultured in large quantities in 300 ml of the above-mentioned L-broth, and the cultured bacteria cells were purified to obtain the DNA (120 µg from #8-7-7 and 60 µg from #9-12-16).

The thus obtained DNA were then subjected to a restriction enzyme treatment and the restriction enzyme map was formed. According to the analysis of this restriction map, it was confirmed that the plasmid was contained, in which on the downstream side of a promoter, an operator and Shine-Dalgarno (SD) sequence (ribosome combining site) of the pUC118 lac gene an exogenote, i.e., the first half portion (containing the section of from the initiation codon to the EcoRI cut position in the approximate center of the structural gene) of the rat pol-β gene was joined to the position of the initiation codon in the first gene (β-galactosidase gene) of the lac gene.

(5)　Introduction of pUC9-10S fragment

[Process of Fig. 1 (c)]

One µg of each purified DNA produced from #8-7-7 and #9-12-16 prepared in the preceding paragraph (4) was cut by the restriction enzyme EcoRI and was further subjected to a phosphatase treatment.

Next, pUC9-10S was prepared in accordance with the method described in the above-mentioned literature of Matsukage, who was one of the present inventors, and Zmudzka et al, and 0.2 µg of EcoRI cut fragment (770 bp coding for the approximate half portion S in Fig. 1 on the side of the C-terminal side of the pol-β) was obtained from the pUC9-10S by the same procedure as in the preceding paragraph (1).

Here, the purified DNA which had undergone the phophatase treatment was linked to the above-mentioned fragment (770 bp) in the similar manner by the use of $T_4$ ligase.

Each of the resulting reaction products was introduced into E. coli JM 109 strain (made by Takara Shuzo Co., Ltd.),

and the latter was cultured. Afterward, the transformed strains were chosen therefrom on an ampicillin plate.

From each group of colonies showing ampicillin resistance, 12 colonies were sampled, i.e., 24 colonies were sampled in all. Each colony was cultured in the above-mentioned L-broth (1.5 ml), and a minilysate was prepared from the cultured bacteria cells in an ordinary manner. The prepared minilysate was cut by EcoRI, and it was inspected by the same procedure as in the preceding paragraph (1) whether or not the EcoRI cut frangment (770 bp) of pUC9-10S was present therein.

On the basis of this result, 6 colonies each having the fragment (770 bp) were chosen from the colonies transformed with the reaction product prepared by using the DNA derived from #8-7-7, and 6 colonies having the same were similarly chosen from the colonies transformed with the reaction product prepared by using the DNA derived from #9-12-16. After each colony was cultured in the same manner as above, a minilysate was prepared from the cultured bacteria cells, and the insertion direction of the fragment (770 bp) was inspected in the same manner as in the preceding paragraph (1). paragraph (1).

As a result, it was confimred that the recombinant (Fig. 1) in which the frangment (770 bp) was inserted in the desired insertion direction on the downstream side of the DNA sequence coding for the approximate half portion F [obtained through the step of Fig. 1(b)] on the side of the N-terminal side of the pol-β, the defective portion of which had been supplemented, was present in four (#8-7-7-2 and #8-7-7-5 to #8-7-7-7) of the colonies transformed with the reaction product prepared by using the DNA derived from #8-7-7, and the above recombinant was present in one (#9-12-16-23) of the colonies transformed with the reaction product prepared by using the DNA derived from #9-12-16.

(6)  Expression of rat pol-β in E. coli host

Each of #8-7-7-2, #8-7-7-5 to #8-7-7-7 and #9-12-16-23 in which the desired recombinant obtained in the preceding paragraph (5) was contained was cultured in the above-mentioned 2XYT culture medium containing 1.5 ml of ampicillin (50 μ/ml), and when the concentration of the bacteria cells had reached $OD_{600}$ = about 0.2, 15 μl of 0.1 M IPTG (isopropylβ-D-thiogalacto pyranoside, made by Sigma Co., Ltd.) was added to the culture medium, followed by further continuing the cultivation overnight.

Then, 22 μl of 50% TCA (trichloroacetic acid) was added to 200 μl of each culture medium (containing the bacteria cells) and was subjected to a centrifugal separation treatment at 15,000 rpm for 3 minutes, and the resulting supernatant liquid was then removed therefrom. Afterward, about 0.5 ml of acetone was added to the precipitate and was then treated by a centrifuge (15,000 rpm, for 3 minutes), so that the resulting supernatant liquid was removed therefrom, with the result that the remaining TCA in the precipitate was eliminated therefrom.

In a solution containing 0.0625 M tris-HCl, 5% of β-mercaptoethanol, 2% of SDS, 10% of glycerol and 0.1% of Bromophenol Blue, 30 μl of each precipitate thus obtained was dissolved, and half of each solution was used for the analysis by an SDS gel electrophoresis.

As a result, it was confirmed that a 40 KDa protein had been produced from these transformed strains.

In this connection, the colony containing the plasmid in which the fragment (770 bp) obtained in the preceding paragraph (5) was inserted in a reverse direction was cultured by the same procedure as above, and its culture medium was treated and the resulting proteins were analyzed. In this case, an extremely thin band derived from the host alone was merely appreciated by the SDS gel electrophoresis.

In addition, when a change in the 40 KDa protein production with time had been inspected, it was confirmed that the approximate maximum peak of the production appeared about 6 hours after the induction by the addition of IPTG.

Further, when the influence of presence and absence of the IPTG on a cel multiplication rate had been investigated, it was confirmed that there was little difference therebetween. Moreover, it was also confirmed that the 40 KDa protein was nontoxic to the cells.

Out of the transformed strains for producing the 40 KDa protein, two strains (#8-7-7-5 and #8-7-7-6) for providing a high production were chosen, and they were cultured separately. Each group of cultured bacteria cells was then treated in an ordinary manner to prepare a plasmid. Each plasmid was analyzed in accordance with the Maxam-Gilbert method in order to elucidate its DNA sequence, and in consequence, it became definite that the plasmid had the promoter and the operator of the pUC118 lac gene as well as the base sequence in which a structural gene (Fig. 4) [which was composed of 1008 bp (containing one termination codon) and which could code the rat pol-β having the sequence of 335 amino acid residues] was inserted into the position where the initiation codon of β-galactosidase had been present, on the downstream side of the Shine-Dalgarno sequence (ribosome combining site).

These plasmids were named pUC118 pol-β-5 and pUC118 pol-β-6, respectively.

Example 2

(Production of rat pol-β by use of recombinant pUC118 pol-β-5 and its purification)

(1) Cultivation of transformed strain into which recombinant pUC118 pol-β-5 was introduced

The transformed strain #8-7-7-5 (JMpβ5) obtained in the preceding paragraph (6) in Example 1 was shake-cultured at 37°C in one liter of the 2XYT culture medium containing 50 µg/ml of ampicillin used in Example 1.

This transformant JMpβ5 was deposited on November 30, 1987 with the Fermentation Research Institute of the Agency of Industrial Science and Technology, 1-3, Higashi 1 chome Tsukuba-shi, Ibaraki-ken, 305 JAPAN under the Budapest Treaty. Its deposition number is FERM BP-1582.

When it was detected by spectroscopic analysis that the concentration of the bacteria cells had reached an absorbance of about 0.5 at 600 nm, the culture medium was divided into two portions. IPTG was then added to one culture medium so as to bring its concentration to 0.5 mM, and the bacteria were further cultured for 7 hours.

After the completion of the cultivation, the becteria cells were collected by a centrifuge. The amount of the collected cells was about 3 g per liter of the culture medium in both the cases.

Each group of the bacteria cells was suspended in a sample solution for an SDS gel electrophoresis containing 1% of SDS and 5% of 2-mercaptoethanol, and heat treatment was performed at 100°C for 2 minutes. After perfectly dissolved therein, the resulting suspension was then subjected to the SDS-polyacrylamide electrophoresis.

As a result, in both the cases where the IPTG was added and not added, a 40 KDa polypeptide was detected in large quantities, and its amount was greater in the case that the IPTG was added than in the other case. In the bacteria cells obtained in the cultivation under the IPTG addition, a ratio of the 40 KDa polypeptide to the total proteins was as much as 19.3%. On occasion, when the growth of the bacteria had reached a saturation (steady) state, the production of the pol-β was in the vicinity of 19.3% at a maximum even

- 36 -

0293491

under conditions of no IPTG.

The size of this 40 KDa polypeptide was comparable to that of the pol-β of a rat, a fowl or the like.

For reference, there was introduced, into E. coli JM109, the plasmid having the above defective portion in the pol-β structural gene obtained by the Zmudzka et al's method used in the paragraph (1) of Example 1, in order to produce the transformed strain JMpβ2-1, and the latter was then cultured by the same procedure as described above. The resulting bacteria cells were afterward anyalyzed to seek proteins.

As a result, the 40 KDa polypeptide, which had been confirmed in the cultivation of the above-mentioned strain JMpβ5, was not found anywhere in this reference experiment.

Apart from the foregoing, E. coli JM109 was cultured in like manner, and the resulting becterial cells were analyzed to seek proteins.

As a result, in this cultivation, the 40 KDa polypeptide, which had been confirmed in the cultivation of the above-mentioned JMpβ5, was not found anywhere, either.

(2) Preparation of bacteria cells extract and measurement of pol-β activity in extract

The respective groups of the bacteria cells obtained in the preceding paragraph (1) were separately treated as follows:

In the first place, each group of bacteria cells was washed with TME [50 mM tris-HCl buffer solution (pH 7.6) containing 10 mM of MgCl$_2$ and 1 mM of EDTA] and was then stored at -80°C.

Next, the washed bacteria cells were suspended in a solution [containing 50 mM of tris-HCl (pH 7.6), 0.1 mM of EDTA, 1 mM of dithiothreitol (DTT), 10% of glycerin, 0.5 M of KCl and 1 mM of phenylmethylsulfonyl fluoride (PMSF)] for extraction in an amount five time as much as that of the bacteria cells, and ultrasonic wave was then applied to the solution for 1 minute in order to destroy the bacteria cells. Further, the solution was treated by means of a centrifuge (12,000 xg, for 20 minutes) to remove the precipitate, thereby obtaining a crude extract of the cells.

Next, for the two kinds of crude extracts thus obtained, the pol-β activity was measured by the method (J. Biol. Chem. 255, p. 9942-9948, 1980) suggested by Yamaguchi, which is one of the present inventors, et al. Here, 1 unit of the activity is defined as an enzyme amount necessary to polymerize 1 nmol of dNTP for 1 hour. The results are set forth in Table 1.

For reference, the crude extract was prepared in like manner by using the JMpβ2-1 cultured in the preceding paragraph (1) and E. coli JM109, and the activity of the pol-β was then measured. The results are also set forth in

Table 1 together.

## TABLE 1

| Strain | IPTG | Activity of DNA Polymerase in Crude Extract (units/µℓ) |
|---|---|---|
| JM 109 | − | 6.2 |
|  | + | 8.0 |
| JMpβ 2-1 (Δ17) | − | 12.3 |
|  | + | 15.2 |
| JMpβ5 | − | 34.9 |
|  | + | 152.2 |

The results indicate that the JMpβ5 inducted with the IPTG has the particularly high activity, which is about 20 time as much as that of the JM109.

In this connection, most of the JM109 activity is considered to be attributable to DNA polymerase I.

The specific activity of the JMpβ5 was $6 \times 10^5$ units per milligram of proteins in terms of 40 KDa polypeptide.

(3) Purification of pol-β from crude extract and inspection of properties thereof

The two kinds of crude extracts obtained from the culture of JMpβ5 in the preceding paragraph (2) were treated as follows:

First, a PC solution [50 mM tris-HCl buffer solution (pH 7.6) containing 0.1 mM of EDTA, 1 mM of DTT and 10% of glycerin] was added to the crude extract so as to dilute the latter about 1.5 times.

A KCl concentration in the dilute crude extract was adjusted to 0.4 M, and the extract was then caused to pass through a column filled with a DEAE cellulose (made by Pharmacia Co., Ltd.) in order to adsorb the nucleic acid components present in the dilute crude extract by the DEAE cellulose, thereby removing these components from the dilute crude extract.

To the fraction (containing rat pol-$\beta$) of the solution which had not been adsorbed on the column, the PC solution having the above-mentioned composition was added so as to dilute the fraction 2 to 3 times, and a KCl concentration in the solution was then adjusted to 0.2 M.

Next, the column filled with cellulose phosphate (made by Sigma Co., Ltd.) was treated with the PC solution containing 0.2 M of KCl so as to equilibrate the cellulose phosphate therein. Afterward, the above adjusted solution was caused to pass through this column, and the latter was then washed well with the PC solution containing 0.2 M of KCl.

By this operation, most of the rat pol-$\beta$ DNA polymerase of the present invention was adsorbed on the column. In this step, the DNA polymerase I and the like derived from

0293491

the host cells were not adsorbed on the column but eluded out, and therefore the desired rat pol-β could be separated therefrom.

The protein adsorbed on the column could be eluted by the utilization of the straight concentration gradient of 0.2 to 0.7 M KCl, and the single peak of the rat pol-β activity could be obtained in the vicinity of about 0.4 M KCl. The fractions having the high rat pol-β activity were collected and then diluted with the PC solution having the above-mentioned composition. Afterward, a KCl concentration and a glycerin concentration in the solution were adjusted to 0.15 M and 20%, respectively.

Next, the thus adjusted solution was caused to pass through the DNA cellulose column made from a modified calf thymus DNA (made by Washington Inc.) in accordance with the above-mentioned Rithoman's method. The column was then washed sufficiently with the PC solution, and the absorbed rat pol-β was eluted from the column with a 0.6 M KCl-PC solution (containing 20% of glycerin). At this time, a single peak which was common to the rat pol-β activity and the protein production could be obtained, and the fraction of this single peak was recovered, thereby obtaining the purified DNA polymerase product.

A protein distribution state in the respective steps of the above purification was traced by an SDS polyacrylamide electrophoresis using a solvent system (a tris buffer solution) of 10% of gel (Laemmli, U.K., Nature, 227, p. 680-685, 1970). In the crude extract treatment step, 6 main thick bands inclusive of the band corresponding to 40 KDa appeared; in the DEAE cellulose treatment step, 6 main thick bands inclusive of the band corresponding to 40 KDa appeared; in the cellulose phosphate treatment step, the single thick band corresponding to 40 KDa appeared; and in the DNA cellulose treatment step, the single thick band corresponding to 40 KDa appeared. This result was common to the two kinds of crude extracts.

The purified porduct which was finally obtained was indicative of a single band (40 KDa) in the polyacrylamide electrophoresis, and its pol-β specific activity was $7.5 \times 10^5$ units/mg, when measured by the method described in the preceding paragraph (2).

On the other hand, a column (1.5 x 50 cm) filled with Sephadex G150 was prepared and then equilibrated with a 0.15 M KCl-PC solution. Afterward, the purified product (fraction eluted with the 0.7 M KCl-PC solution) obtained through the treatment by the use of the above-mentioned DNA cellulose column was injected into th equilibrated column and was then eluted with the 0.15 M KCl-PC solution. The eluates were separately received, and for the respective fractions, pol-β activities were measured. Afterward, the fractions having the high pol-β activities were collected and injected into the DNA cellulose column (0.6 x 5 cm), and the latter was then washed well with the 0.15 M KCl-PC solution. The components adsorbed on the column were eluted with 15 ml of 0.15-0.7 M KCl by the utilization of its straight concentration gradient (in the PC solution), and the fractions having the high pol-β activity were collected, thereby obtaining the purified product. The concentration of KCl at the time of the eluation was 0.4 M. In the purified product obtained in this way, the purity of the poly-β was as high as 98 to 99%.

When the above purified product was compared with the pol-β, which had been directly extracted from rat cells and then purified, in point of the results of the polyacrylamide electrophoresis, both of them had the size of the 40 KDa, and any difference therebetween was not be perceptible.

Next, the purified product was analyzed in accordance with the western blotting method.

In the first place, the protein contained in the 40 KDa band of the above purified product which had been obtained by the SDS polyacrylamide electrophoresis was transferred onto a nitrocellulose film by the use of a blotting device (made by Bio-Rad Co., Ltd.). Afterward, blotting was carried out at 2,000V for 15 hours in a blotting solution comprising 25 mM of tris/192 mM glycine (pH 8.3) and 20% methanol.

The film was then blocked with TBS [50 mM of tris-HCl (pH 8.3) and 150 mM of NaCl] containing 20% of a bovine fetal serum, and it was reacted with a 500 times-diluted antifowl pol-β rabbit antibody at room temperature for 2 hours and was further reacted with antirabbit IgG goat antibody combining with peroxidase at room temperature for 2 hours.

In the last place, the film was washed and then dipped into TBS containing 0.05% of 4-chloronaphthol, 0.015% of $H_2O_2$ and 16.5% of methanol, and an antigen polypeptide was identified by a colorific reaction.

As a result, the polypeptide obtained from the purified product and the antifowl pol-β rabbit antibody crossed over.

From the above results, it was confirmed that the above purified product had the rat pol-β activity. In addition, it was appreciated that the enzyme activity of the purified product was similar to that of a natural one and that the specific activity thereof was as high as that of the natural one.

Next, the nuclease activity of the above purified product was measured as follows:

First, in order to measure a $3' \to 5'$ exonuclease activity, the plasmid pM1-1 (which was prepared by the present inventors themselves) was cut by the restiction enzymes HindIII and SclI, and a fragment of 2347 bp was obtained by an agarose gel electrophoresis. Then, the 3'
terminal of the fragment was marked by the use of $^{32}$P-dCTP
and klenow enzyme.

Afterward, a reaction solution was prepared which was
composed of 10 mM of tris-HCl (pH 7.5), 7 mM of MgCl$_2$, 20 mM
of NaCl, 7 mM of 2-mercaptoethanol, 100 µg/ml of gelatin, a
marker DNA of $10^5$ cpm and 60 to 75 units of the purified
product, and reaction was then performed at 37°C.

As the reaction progressed, the solution was sampled
(10 µl) periodically, and the samples were analyzed by a
0.5% agarose gel electrophoresis and an autoradiogram, so
that the 3' → 5' exonuclease activity was sought on the
basis of the increase in $^{32}$P from the marked DNA fragment.

As a result, the 3' → 5' exonuclease activity was not
perceptible in the purified product.

When a similar measurement was carried out by the use
of klenow enzyme, the activity was appreciated.

Further, the endonuclease activity of the purified
product was sought on the basis of whether or not the closed
chain DNA (type I) of the plasmid pUC19 DNA was converted
into the opened chain (type II) by cutting the DNA chain.

That is, the reaction solution having the same
composition as in the measurement step of the exonuclease
activity was prepared with the exception that the marked DNA
fragment was replaced with 2 µg of pUC19 DNA (made by Takara

- 45 -

0293491

Shuzo Co., Ltd.; type I was from 80 to 90%) and that the enzyme amount was from 80 to 130 units. The thus prepared reaction solution was maintained at 37°C to advance a reaction, and sampling was performed periodically. The samples were subjected to a 0.6% agarose gel electrophoresis and were dyed with ethidium bromide, and the decrease in the type I and the increase in the type II were traced.

In consequence, with regard to the purified product obtained in this example, any endonuclease activity was not perceptible. The identical measurement was carried out by the use of the klenow enzyme, but any activity was not appreciated, either.

## INDUSTRIAL APPLICABILITY

According to the present application, a DNA polymerase derived from an animal such as rat can be expressed in host cells by a genetic engineering technique and the pure DNA polymerase can be produced in large amounts.

In addition, although the DNA polymerase I derived from E. coli and the klenow enzyme which have often heretofore been used possess the nuclease activity, the DNA polymerase β obtained by the present invention has no nuclease activity. Therefore, in the DNA synthesis by the use of the DNA polymerase which the present invention provides, the 3' terminal thereof can be completed. In consequence, the DNA polymerase of the present invention can be directly applied to the DNA synthesis process in which any nuclease activity is not required or is not desired, and it can be mass-produced in the pure form. It is fair to say that the present invention can sufficiently meet the increasing demand for the DNA polymerase.

Further, the DNA polymerase obtained in accordance with the present invention is also useful as a component of, for example,

(a) an enzyme agent for the recombination, for example, to sequence a DNA and

(b) an enzyme agent for the reaction for forming a double chain, when a mutation is caused at an optional site on a DNA, i.e., at the time of the so-called site specific mutation induction.

Accordingly, the DNA polymerase of the present invention is widely and versatilely usable in the protein engineering fields.

In the case that the vector capable of taking the morphology of a single chain DNA is used in the recombinant for DNA polymerase expression into which the DNA sequence for coding the DNA polymerase of the present invention is introduced, the vector can be immediately combined with a synthesized oligonucleotide primer containing a site specific mutation region. Therefore, by the mismatch primer process, the desired double helix DNA can be obtained in which substitution, insertion, deletion or the like has been given. When the thus obtained DNA is introduced into a suitable host, a protein having a new physiological activity can be expressed.

The DNA polymerase which can be mass-produced by the present invention can be effectively utilized as a reagent in, for example, a mutation characteristic test in vitro other than an Ames Test.

This mutation characteristic test is utilized to detect miscoding in synthesizing a complementary DNA to a template DNA by a mutation original material, as the mutation of a phage in the system of E. coli-bacteriophage. This test is different from the Ames Test and can also elucadate the mechanism of the mutation and has a very high sensitivity advantageously. Therefore, the present invention can contribute to the spread of such a useful test.

Since enabling the mass production of the DNA polymerase, the present invention can meet the increased demand for the DNA polymerase which is used as diagnostic and remedial medicines for animal diseases.

On the other hand, it is now known that DNA Ase I which is a kind of DNA lytic enzyme is effective against congestions on a skin, and it is actually used for such a treatment at present. Therefore, the DNA polymerase which is a kind of DNA synthetic enzyme may be inversely concerned with the expression mechanism of various diseases. In addition, it might be discovered that the DNA polymerase is effective against various diseases such as hereditary sicknesses and the like, and for the sake of diagnoses and treatments for these diseases, the mass production of the human DNA polymerase must be assured. As understood from the foregoing, the present invention can provide the technique which can meet such a demand sufficiently.

CLAIMS

1. A peptide comprising the following amino acid sequence:

Met Ser Lys Arg Lys Ala Pro Gln Glu Thr Leu Asn Gly Gly Ile Thr Asp Met Leu Val Glu Leu Ala Asn Phe Glu Lys Asn Val Ser Gln Ala Ile His Lys Tyr Asn Ala Tyr Arg Lys Ala Ala Ser Val Ile Ala Lys Tyr Pro His Lys Ile Lys Ser Gly Ala Glu Ala Lys Lys Leu Pro Gly Val Gly Thr Lys Ile Ala Glu Lys Ile Asp Glu Phe Leu Ala Thr Gly Lys Leu Arg Lys Leu Glu Lys Ile Arg Gln Asp Asp Thr Ser Ser Ser Ile Asn Phe Leu Thr Arg Val Thr Gly Ile Gly Pro Ser Ala Ala Arg Lys Leu Val Asp Glu Gly Ile Lys Thr Leu Glu Asp Leu Arg Lys Asn Glu Asp Lys Leu Asn His His Gln Arg Ile Gly Leu Lys Tyr Phe Glu Asp Phe Glu Lys Arg Ile Pro Arg Glu Glu Met Leu Gln Met Gln Asp Ile Val Leu Asn Glu Val Lys Lys Leu Asp Pro Glu Tyr Ile Ala Thr Val Cys Gly Ser Phe Arg Arg Gly Ala Glu Ser Ser Gly Asp Met Asp Val Leu Leu Thr His Pro Asn Phe Thr Ser Glu Ser Ser Lys Gln Pro Lys Leu Leu His Arg Val Val Glu Gln Leu Gln Lys Val Arg Phe Ile Thr Asp Thr Arg Ser Lys Gly Glu Thr Lys Phe Met Gly Val Cys Gln Leu Pro Ser Glu Asn Asp Glu Asn Glu Tyr Pro His Arg Arg Ile Asp Ile Arg Leu Ile Pro Lys Asp Gln Tyr Tyr Cys Gly Val Leu Tyr Phe Thr Gly Ser Asp Ile Phe Asn Lys Asn Met Arg Ala His Ala Leu Glu Lys Gly Phe Thr Ile Asn Glu Tyr Thr Ile Arg Pro Leu Gly Val Thr Gly Val Ala Gly Glu Pro Leu Pro Val Asp

Ser Glu Gln Asp Ile Phe Asp Tyr Ile Gln Trp Arg Tyr Arg Glu

Pro Lys Asp Arg Ser Glu.


2. A DNA comprising a DNA sequence for coding a DNA polymerase produced by an animal.


3. A DNA according to Claim 2 wherein said DNA polymerase produced by the animal has said amino acid sequence described in Claim 1.


4. A DNA according to Claim 2 wherein said DNA sequence for coding said DNA polymerase produced by the animal is as follows:

ATGAGCAAAC GCAAGGCCCC GCAGGAGACC CTCAACGGCG GCATCACGGA

CATGCTCGTG GAACTCGCAA ACTTTGAGAA GAACGTGAGC CAGGCGATCC

ACAAGTACAA TGCATACAGA AAAGCAGCAT CTGTGATAGC AAAGTACCCA

CACAAAATCA GAGTGGAGC AGAAGCTAAG AAATTGCCAG GAGTAGGAAC

AAAAATTGCT GAAAAGATTG ATGAATTTTT AGCAACTGGA AAATTGCGTA

AACTGGAAAA GATTCGTCAG GATGATACAA GTTCATCCAT CAACTTCCTG

ACTCGAGTTA CTGGCATCGG GCCATCTGCT GCAAGGAAGC TTGTAGATGA

AGGAATTAAA ACATTAGAAG ATCTCAGGAA AAATGAAGAT AAATTGAACC

ACCATCAGCG AATTGGGCTG AAATATTTTG AGGACTTTGA AAAGAGAATT

CCTCGTGAGG AGATGCTGCA AATGCAGGAC ATTGTTCTTA ATGAAGTTAA

AAAGCTGGAT CCCGAGTACA TCGCTACAGT CTGCGGCAGT TTCCGAAGAG

GCGCAGAGTC CAGTGGAGAT ATGGACGTTC TGCTGACCCA CCCAAACTTC

ACGTCAGAAT CAAGCAAACA GCCAAAGTTG TTACATCGTG TTGTGGAACA

GTTACAAAAA GTCCGTTTCA TTACAGATAC TCGTTCAAAG GGTGAGACAA

AGTTCATGGG TGTTTGCCAG CTTCCCAGCG AGAATGATGA AAACGAATAT

CCACACAGGA GAATCGATAT CAGGTTGATC CCCAAAGATC AGTACTACTG

TGGTGTTCTC TACTTCACTG GAAGTGACAT CTTTAATAAG AATATGAGAG

CGCATGCCCT GGAAAAGGGC TTCACAATCA ATGAGTACAC CATCCGCCCC

CTGGGGGTCA CTGGGGTTGC TGGGGAGCCC CTTCCGGTGG ACAGCGAGCA

GGACATTTTC GATTACATCC AGTGGCGCTA CCGGGAGCCC AAGGACAGGA

GTGAATGA.

5. A recombinant plasmid which comprises a vector having at least a section which can be replicated in a host cell, and a DNA fragment containing a DNA sequence for coding a DNA polymerase produced by the animal combining with said vector.

6. A recombinant plasmid according to Claim 5 wherein said DNA polymerase produced by the animal has said amino acid sequence described in Claim 1.

7. A recombinant plasmid according to Claim 5 wherein said DNA sequence for coding said DNA polymerase produced by the animal is as shown in Claim 4.

8. A recombinant plasmid according to any one of Claims 5 to 7 wherein said vector has the feature that it takes the morphology of a single chain, when a phage is added thereto.

9. A recombinant plasmid according to Claim 5 wherein said vector is a DNA fragment derived from plasmid pUC118, and said DNA sequence for coding said DNA polymerase is present on the downstream side of the promoter of a fractose operon of said DNA fragment.

10. A microorganism or a cell transformed by said recombinant plasmid described in any one of Claims 5 to 8.

11. A microorganism according to Claim 10 wherein said microorganism is Escherichia coli.

12. A method for producing said DNA polymerase by culturing said transformed miroorganism or cell described in Claim 10 or 11.

13. A method for separating/purifying a DNA polymerase from a mixture containing said DNA polymerase produced by an animal cell which comprises the steps of:

(a) bringing said mixture into contact with an anion exchange carrier;

- 53 -

0293491

(b) bringing, into contact with an acidic carrier, the fraction of said mixture which has not been adsorbed on said anion exchange carrier;

(c) fractionating said fraction adsorbed on said acidic carrier by the utilization of a difference between salt concentrations in eluates in order to separate said fraction having a DNA polymerase activity; and

(d) treating said fraction having said DNA polymerase activity with an affinity chromatography to purify said DNA polymerase.

(14) A method for separating/purifying a DNA polymerase according to Claim 11 wherein said mixture contains said DNA polymerase produced by a microorganism or an animal cell transformed by introducing thereinto a DNA sequence for coding said DNA polymerase produced by an animal cell.

M/3071.7

**0293491**

INTERNATIONAL FORM

BUDAPEST TREATY ON THE INTERNATIONAL RECOGNITION OF THE DEPOSIT OF MICROORGANISMS FOR THE PURPOSES OF PATENT PROCEDURE

RECEIPT IN THE CASE OF AN ORIGINAL DEPOSIT
issued pursuant to Rule 7.1 by the INTERNATIONAL DEPOSITARY AUTHORITY identified at the bottom of this page.

To    Mr. Haruo SAWAMURA, President
Mitsui Toatsu Chemicals, Incorporated
2-5, Kasumigaseki 3-chome, Chiyoda-ku, Tokyo

---

**I. IDENTIFICATION OF THE MICROORGANISM**

Identification reference given by the DEPOSITOR:

Accession number given by the INTERNATIONAL DEPOSITARY  AUTHORITY: 1582

JMp β5

(FERM BP-1582)

**II. SCIENTIFIC DESCRIPTION AND/OR PROPOSED TAXONOMIC DESIGNATION**

The microorganism identified under I above was accompanied by:

[X]    a scientific description

[X]    a proposed taxonomic designation

**III. RECEIPT AND ACCEPTANCE**

This International Depositary Authority accepts the microorganism identified under I above, which was received by it on November 30, 1987.

**IV. INTERNATIONAL DEPOSITARY AUTHORITY**

NAME    :   Fermentation Research Institute
Agency of Industrial Science and Technology

_____ (Signature) _____
Akio SATO, Dr.    DIRECTOR GENERAL

ADDRESS: 1-3, Higashi 1-chome, Tsukuba-shi, Ibaraki-ken, 305, JAPAN

November 30, 1987

F I G . 1

pUC118 → pUC9-10F → (a) → pUC118+F → (b) → pUC118+F' → pUC9-10S → (c) → pUC118+F'+S

MCS: EcoRI, SacI, KpnI, SmaI, BamHI, XbaI, SalI, PstI, SphI, HindⅢ

F I G . 2

EcoRI  XhoI  BglⅡ  EcoRI
pol β·F  β-gal
β-gal  approx.210b  HindⅢ
approx.140b

0293491

# F I G.3

(a) 5′—C[AGGA]AACAGCT[ATGACCATGATTACGAATTCGAGCTC]-- pUC118

SD    β-galgene    MCS
EcoRI    SacI

(b) 5′—C[AGGA]AACAGCT[ATGACCATGATTACGAATTCGCAAGGCCCCG]-- pUC118+F

β-galgene    EcoRI    Rat pol βcDNA

(c) 5′—C[AGGA]AACAGCT[ATG]ᴬ  T.T[CGCAAGGCCCCG]-- -3′  pUC118+F

3′  CCTTTGTCGATACTCGTTTGCGTTCCGGGG  5′  Mutagenic primer (30-mer)

(d) 5′—C[AGGA]AACAGCT[ATGAGCAAACGCAAGGCCCCG]  pUC118+F′

cDNA

**F I G. 4A**

```
  1 ATG AGC AAA CGC AAG GCC CCG CAG GAG ACC     30
  1 Met Ser Lys Arg Lys Ala Pro Gln Glu Thr     10

 31 CTC AAC GGC GGC ATC ACG GAC ATG CTC GTG     60
 11 Leu Asn Gly Gly Ile Thr Asp Met Leu Val     20

 61 GAA CTC GCA AAC TTT GAG AAG AAC GTG AGC     90
 21 Glu Leu Ala Asn Phe Glu Lys Asn Val Ser     30

 91 CAG GCG ATC CAC AAG TAC AAT GCA TAC AGA    120
 31 Gln Ala Ile His Lys Tyr Asn Ala Tyr Arg     40

121 AAA GCA GCA TCT GTG ATA GCA AAG TAC CCA    150
 41 Lys Ala Ala Ser Val Ile Ala Lys Tyr Pro     50

151 CAC AAA ATC AAG AGT GGA GCA GAA GCT AAG    180
 51 His Lys Ile Lys Ser Gly Ala Glu Ala Lys     60

181 AAA TTG CCA GGA GTA GGA ACA AAA ATT GCT    210
 61 Lys Leu Pro Gly Val Gly Thr Lys Ile Ala     70

211 GAA AAG ATT GAT GAA TTT TTA GCA ACT GGA    240
 71 Glu Lys Ile Asp Glu Phe Leu Ala Thr Gly     80

241 AAA TTG CGT AAA CTG GAA AAG ATT CGT CAG    270
 81 Lys Leu Arg Lys Leu Glu Lys Ile Arg Gln     90
```

3/6

0293491

**F I G. 4B**

```
271 GAT GAT ACA AGT TCA TCC ATC AAC TTC CTG 300
 91 Asp Asp Thr Ser Ser Ser Ile Asn Phe Leu 100

301 ACT CGA GTT ACT GGC ATC GGG CCA TCT GCT 330
101 Thr Arg Val Thr Gly Ile Gly Pro Ser Ala 110

331 GCA AGG AAG CTT GTA GAT GAA GGA ATT AAA 360
111 Ala Arg Lys Leu Val Asp Glu Gly Ile Lys 120

361 ACA TTA GAA GAT CTC AGG AAA AAT GAA GAT 390
121 Thr Leu Glu Asp Leu Arg Lys Asn Glu Asp 130

391 AAA TTG AAC CAC CAT CAG CGA ATT GGG CTG 420
131 Lys Leu Asn His His Gln Arg Ile Gly Leu 140

421 AAA TAT TTT GAG GAC TTT GAA AAG AGA ATT 450
141 Lys Tyr Phe Glu Asp Phe Glu Lys Arg Ile 150

451 CCT CGT GAG GAG ATG CTG CAA ATG CAG GAC 480
151 Pro Arg Glu Glu Met Leu Gln Met Gln Asp 160

481 ATT GTT CTT AAT GAA GTT AAA AAG CTG GAT 510
161 Ile Val Leu Asn Glu Val Lys Lys Leu Asp 170

511 CCC GAG TAC ATC GCT ACA GTC TGC GGC AGT 540
171 Pro Glu Tyr Ile Ala Thr Val Cys Gly Ser 180
```

0293491

FIG.4C

541 TTC CGA AGA GGC GCA GAG TCC AGT GGA GAT 570
181 Phe Arg Arg Gly Ala Glu Ser Ser Gly Asp 190

571 ATG GAC GTT CTG CTG ACC CAC CCA AAC TTC 600
191 Met Asp Val Leu Leu Thr His Pro Asn Phe 200

601 ACG TCA GAA TCA AGC AAA CAG CCA AAG TTG 630
201 Thr Ser Glu Ser Ser Lys Gln Pro Lys Leu 210

631 TTA CAT CGT GTT GTG GAA CAG TTA CAA AAA 660
211 Leu His Arg Val Val Glu Gln Leu Gln Lys 220

661 GTC CGT TTC ATT ACA GAT ACT CGT TCA AAG 690
221 Val Arg Phe Ile Thr Asp Thr Arg Ser Lys 230

691 GGT GAG ACA AAG TTC ATG GGT GTT TGC CAG 720
231 Gly Glu Thr Lys Phe Met Gly Val Cys Gln 240

721 CTT CCC AGC GAG AAT GAT GAA AAC GAA TAT 750
241 Leu Pro Ser Glu Asn Asp Glu Asn Glu Tyr 250

751 CCA CAC AGG AGA ATC GAT ATC AGG TTG ATC 780
251 Pro His Arg Arg Ile Asp Ile Arg Leu Ile 260

0293491

**F I G. 4D**

```
781 CCC AAA GAT CAG TAC TAC TGT GGT GTT CTC  810
261 Pro Lys Asp Gln Tyr Tyr Cys Gly Val Leu  270

811 TAC TTC ACT GGA AGT GAC ATC TTT AAT AAG  140
271 Tyr Phe Thr Gly Ser Asp Ile Phe Asn Lys  280

841 AAT ATG AGA GCG CAT GCC CTG GAA AAG GGC  870
281 Asn Met Arg Ala His Ala Leu Glu Lys Gly  290

871 TTC ACA ATC AAT GAG TAC ACC ATC CGC CCC  900
291 Phe Thr Ile Asn Glu Tyr Thr Ile Arg Pro  300

901 CTG GGG GTC ACT GGG GTT GCT GGG GAG CCC  930
301 Leu Gly Val Thr Gly Val Ala Gly Glu Pro  310

931 CTT CCG GTG GAC AGC GAG CAG GAC ATT TTC  960
311 Leu Pro Val Asp Ser Glu Gln Asp Ile Phe  320

961 GAT TAC ATC CAG TGG CGC TAC CGG GAG CCC  990
321 Asp Tyr Ile Gln Trp Arg Tyr Arg Glu Pro  330

991 AAG GAC AGG AGT GAA TGA      1008
331 Lys Asp Arg Ser Glu ****      335
```

0293491

0293491

# INTERNATIONAL SEARCH REPORT

International Application No PCT/JP87/00938

**I. CLASSIFICATION OF SUBJECT MATTER** (If several classification symbols apply, indicate all) [3]

According to International Patent Classification (IPC) or to both National Classification and IPC

Int.Cl$^4$    C12N9/12, 15/00

**II. FIELDS SEARCHED**

| Minimum Documentation Searched [4] | |
|---|---|
| Classification System | Classification Symbols |
| IPC | C12N9/12, 15/00 |

| Documentation Searched other than Minimum Documentation to the Extent that such Documents are Included in the Fields Searched [5] |
|---|
| COMPUTER SEARCH, BIOSIS PREVIEWS |

**III. DOCUMENTS CONSIDERED TO BE RELEVANT** [14]

| Category [*] | Citation of Document, [16] with indication, where appropriate, of the relevant passages [17] | Relevant to Claim No. [18] |
|---|---|---|
| X | Archives of Biochemistry and Biophysis, Vol. 198, No. 2, (1979), A. Weissbach [The Functional Roles of Mammalian DNA Polymerase], P.386-396 | 1 |
| X, Y | Biochimica et Biophysica Acta, Vol. 383, (1975), A. Matsukage, et al [Differential Sensitivity of Low Molecular Weight DNA Polymerase to Sulfhydryl-Blocking Reagents], P.338-343 | 1, 13, 14 |
| X, Y | The Journal of Biological Chemistry, Vol. 256, No. 6, (1981), K. Tanabe, et al [Structural Homology of DNA Polymerase β from Various Mammalian Cells], P. 3098-3102 | 1, 13, 14 |
| A | Proceedings of the National Academy of Sciences of the United States of America, Vol. 83, No. 14, (1986), B.Z. Zmudzka, et al [Structure of rat DNA Polymerase β revealed by partial amino | 2-12 |

* Special categories of cited documents: [16]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search [2] | Date of Mailing of this International Search Report [2] |
|---|---|
| February 8, 1988 (08. 02. 88) | February 22, 1988 (22. 02. 88) |
| International Searching Authority [1] | Signature of Authorized Officer [10] |
| Japanese Patent Office | |

Form PCT/ISA/210 (second sheet) (October 1977)

FURTHER INFORMATION CONTINUED FROM THE SECOND SHEET

| | acid sequencing and cDNA cloning], p. 5106-5110 | |
|---|---|---|
| P | Molecular and Cellular Biology, Vol. 7, No. 5, (1987), M. Yamaguchi, et al [Murine DNA Polymerase β Gene: Mapping of Transcription Initiation Sites and the Nucleotide Sequence of the Putative Promoter Region], p.2012-2018 | 2-12 |
| P | The Journal of Biological Chemistry, Vol. 262, No. 19, (1987), A. Matsukage, et al [Homology between Mammalian DNA Polymerase β and Terminal Deoxynucleotidyltransferase], p.8960-8962 | 2-12 |

**V.☐ OBSERVATIONS WHERE CERTAIN CLAIMS WERE FOUND UNSEARCHABLE [10]**

This international search report has not been established in respect of certain claims under Article 17(2) (a) for the following reasons:

1.☐ Claim numbers_____, because they relate to subject matter [12] not required to be searched by this Authority, namely:

2.☐ Claim numbers_____, because they relate to parts of the international application that do not comply with the prescribed requirements to such an extent that no meaningful international search can be carried out [13], specifically:

**VI.☐ OBSERVATIONS WHERE UNITY OF INVENTION IS LACKING [11]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest
☐ The additional search fees were accompanied by applicant's protest.
☐ No protest accompanied the payment of additional search fees.